# FASCICULE DE BREVET EUROPEEN

(11) **EP 3 353 285 B1**
(45) Date de publication et mention de la délivrance du brevet: **22.07.2020**
(21) Numéro de dépôt: 16781517.4
(22) Date de dépôt: 20.09.2016
(51) Int. Cl.: C12N 1/16, C12N 1/18, C12G 1/02, C12R 1/85, C12R 1/865

(54) **SOUCHES DE LEVURE POUR BOISSONS FERMENTEES ET NOTAMMENT POUR LE VIN**
HEFESTÄMME FÜR FERMENTIERTE GETRÄNKE UND INSBESONDERE FÜR WEIN
YEAST STRAINS FOR FERMENTED BEVERAGES AND PARTICULARLY FOR WINE

(30) Priorité: 21.09.2015 FR 1558875
(43) Date de publication de la demande: 01.08.2018
(73) Titulaire: Lesaffre et Compagnie, 75001 Paris (FR)
(72) Inventeur: DORIGNAC, Etienne, 09270 Mazeres (FR); QUIPOURT, Anne-Dominique, 59700 Marc En Baroeul (FR); TBAIKHI, Annie, 59118 Wambrechies (FR)
(74) Mandataire: Plasseraud IP
(86) Numéro de dépôt international: PCT/FR2016/052385
(87) Numéro de publication internationale: WO 2017/051114

(56) Documents cités:
- Luc Laffort: "Laffort Vintage 2015", , 7 juillet 2015 (2015-07-07), page 102PP, XP002758086, Extrait de l'Internet: URL:http://www.cellartek.com/application/f iles/4514/3647/7514/Laffort_US_HarvestCata logue_2015.compressed_2.pdf [extrait le 2016-05-26]

## Description

### Domaine technique de l'invention

La présente invention concerne des souches de levures à destination des boissons fermentées ainsi que des méthodes de sélection desdites souches. La présente demande concerne en outre l'utilisation des levures issues de ces souches pour la production de vin.

### Arrière-plan technologique

La fermentation est l'une des étapes essentielles à la production de vin durant laquelle les sucres présents dans le moût de raisin vont se transformer en alcool via l'action de microorganismes tels que les levures.

Différentes souches de levure sont utilisées selon le type de vin produit. La souche de levure *Saccharomyces* S1, de la demanderesse, a une capacité de fermenter régulièrement mais très lentement : elle est donc particulièrement adaptée aux vins rouges de qualité premium issus d'un long processus de macération/extraction afin de tirer parti de la richesse en polyphénols (anthocyanes, tannins) de cépages noirs très colorés et très tanniques.

Malheureusement, cette souche présente de grandes difficultés à fermenter des moûts à potentiel alcoolique supérieur à 14% v/v en présence d'une quantité normale voire même élevée d'azote assimilable dans le moût. Or, ces conditions sont courantes pour des vins rouges voulus « de garde », qui par nature ont un haut potentiel alcoolique et sont issus de raisins surmûris donnant des moûts pauvres en azote assimilable. Dans ces conditions, il y a un donc un fort risque d'arrêt prématuré de la fermentation, ce qui est préjudiciable à la production viticole.

Il serait donc particulièrement avantageux de développer des moyens permettant d'améliorer les capacités de fermentation de la souche de levure S1, ce afin d'accroître son intérêt d'utilisation en vinification.

L'amélioration des levures peut être réalisée soit à l'aide des techniques génétiques classiques soit à l'aide de techniques de génétique moléculaire. Cependant, le marché de la vinification est très réfractaire à l'usage de microorganismes génétiquement modifiés (OGM).

La brochure publicitaire du groupe Laffort® « Laffort® Vintage 2015 » (URL : https://www.cellartek.com/application/files/4514/3647/7514/Laffort _US_HarvestCatalo gue_2015.compressed_2.pdf) propose à la vente plusieurs souches de levures pour une utilisation dans la fermentation du vin. Les souches de levures décrites dans ce document sont présentées comme présentant certaines capacités utiles pour la vinification.

### Invention

Ainsi, les inventeurs ont déterminé qu'il était possible d'obtenir une souche particulièrement intéressante en vinification de cépages noirs à taux élevés en tanins par hybridation de la souche S1 avec une souche de levure ayant la capacité de fermenter rapidement une grande quantité de sucres en présence d'une faible quantité d'azote et à basse température : la souche de levure de l'espèce *Saccharomyces bayanus* S2 appartenant à la demanderesse.

Grâce à ce processus d'hybridation, les inventeurs ont produit et sélectionné plusieurs souches qui présentent une très bonne cinétique de fermentation et une résistance à un degré alcoolique supérieur à 15% V/V et une capacité de fermentation en présence d'une faible quantité d'azote assimilable. Ces souches trouvent tout particulièrement leur intérêt dans la vinification de moûts pauvres en azote assimilable qui nécessitent une fermentation longue, ce qui est le cas des vins dits « de garde » comme les grands crus de Bordeaux.

Ainsi, selon un premier aspect, l'invention concerne une souche de levure susceptible d'être obtenue par hybridation d'une souche S1 avec une souche S2, ladite souche de levure présentant, selon un test A tel que défini ci-après, les caractéristiques suivantes :
- une cinétique de fermentation de 15 à 22 jours à une température de 24°C; et
- une résistance à un degré alcoolique supérieur ou égal à 15% v/v ; et
- un besoin azoté inférieur ou égal à 200ppm.

Les inventeurs ont également mis au point une méthode permettant d'obtenir et de sélectionner des souches de levure œnologiques issues de l'hybridation d'une souche S1 avec une souche S2.

Ainsi, selon un autre aspect, la présente invention concerne une méthode d'obtention d'une souche de levure œnologique, ladite méthode comprenant les étapes suivantes :
a) hybridation d'une souche de levure S1 avec une souche de levure S2 ;
b) isolement des souches obtenues à l'étape a) ;
c) ensemencement des souches isolées à l'étape b) sur un moût;
d) étude de la cinétique de fermentation desdites souches ; et
e) sélection des souches présentant une cinétique de fermentation au moins 15%, de préférence au moins 30%, plus préférentiellement au moins 40% supérieure à la souche parente S1 et au moins 15%, de préférence au moins 30%, plus préférentiellement au moins 40% inférieure à la souche parente S2 sur le même moût que celui de l'étape c).

L'invention concerne également l'utilisation d'une souche de levure telle que décrite ci-dessus pour la production de boissons fermentées et plus particulièrement de vin.

### Description détaillée de l'invention

Afin d'améliorer les capacités vinicoles de la souche de levure S1, notamment ses capacités de fermentation, les inventeurs ont développé de nouvelles souches particulièrement adaptées à une utilisation vinicole.

Par « caractéristiques de fermentation améliorées », on entend ici des souches de levure qui ont à la fois les avantages apportés pas la souche S1, particulièrement une capacité de fermenter un moût à une vitesse modérée et régulière, sans défauts analytiques tels qu'une production d'acidité volatile moyenne et une faible production de SO₂ et avec des caractéristiques organoleptiques intéressantes telles que l'extraction poussée de polyphénols et la promotion d'arômes fruités mais qui puissent également fermenter rapidement, éventuellement en présence d'une quantité d'azote assimilable modérée et de manière prolongée (même quand le degré d'alcool du moût dépasse 15% v/v).

Les critères de sélection qui ont été retenus ici sont donc les suivants :
- une cinétique de fermentation plus élevée que celle de la souche parente S1 mais tout aussi régulière que celle de cette dernière ; et
- une capacité à fermenter en présence d'une quantité d'azote assimilable modérée ; et
- une résistance à un degré d'alcool élevé (c'est-à-dire supérieur ou égal à 15%).

Ainsi, un premier aspect de l'invention concerne une souche de levure susceptible d'être obtenue par hybridation d'une souche S1 avec une souche S2, ladite souche de levure présentant, selon le test A, les caractéristiques suivantes :
i) une cinétique de fermentation de 15 à 22 jours à une température de 24°C; et
ii) une résistance à un degré alcoolique supérieur ou égal à 15% v/v; et
iii) un besoin azoté inférieur ou égal à 200ppm.

Le « test A » selon la présente invention est un test durant lequel on suit l'activité de fermentation de souches de levure qui sont ensemencées à hauteur de 2.10⁶ UFC/ml sur un moût synthétique standardisé de l'Organisme International de la Vigne et du Vin - OIV (RÉSOLUTION OIV-OENO 370-2012) ajusté à 250g/l de sucres fermentescibles (pour obtenir un alcool potentiel de 15% v/v) avec une concentration initiale en azote de 200ppm en fermentant à une température constante de 24°C.

L'expression « Souche de levure » désigne une population relativement homogène de cellules de levure. Une souche de levure est obtenue à partir d'un clone, un clone étant une population de cellules obtenue à partir d'une seule cellule de levure.

On entend par « souche de levure œnologique » une souche qui est utilisée pour la fermentation du vin.

Par souche de levure ou souche S1, on fait ici référence à la souche de levure de la demanderesse déposée le 9 septembre 2015 à la Collection Nationale de Cultures de Microorganismes (CNCM) -Institut Pasteur, 25 rue du docteur Roux F75724 Paris sous la référence I-5011.

Par souche de levure ou souche S2, on fait ici référence à la souche de levure au nom de la demanderesse déposée le 9 septembre 2015 à la CNCM sous la référence I-5012.

Une « résistance à un degré alcoolique supérieur ou égal à X% v/v» signifie que la souche de levure est capable de survivre et de continuer à fermenter lorsque le titre alcoométrique volumique du milieu est supérieur ou égal à X% v/v.

Le « besoin azoté » d'une souche de levure est défini par la quantité d'azote assimilable minimale qui doit être présente dans le moût pour que ladite souche puisse achever la fermentation (présence de moins de 2g/l de sucres fermentescibles résiduels) sur un moût donné.

La cinétique de fermentation peut être facilement mesurée par différentes techniques connues de l'homme du métier. Par exemple, la cinétique de fermentation peut-être mesurée via un suivi de fermentation par pesée au cours du temps.

L'invention concerne particulièrement deux nouvelles souches de levures telles que décrites ci-dessus : les souches 51-062 et 51-135 déposées le 5 mai 2015 à la CNCM en vertu du traité de Budapest respectivement sous les numéros I-4975 et I-4977

L'invention concerne également une levure issue d'une souche de levure telle que décrite ci-dessus.

Les inventeurs ont également élaboré un processus d'hybridation de la souche S1 avec la souche de *Saccharomyces bayanus* S2, souche qui a la capacité de fermenter rapidement une grande quantité de sucres en présence d'une faible quantité d'azote à basse température. Puis, ils ont mis au point un procédé de sélection permettant d'identifier, parmi les différentes souches issues de cette hybridation, les souches présentant des caractéristiques de fermentations améliorées et donc intéressantes dans la vinification.

Ainsi dans un autre aspect, l'invention concerne une méthode d'obtention d'une souche de levure œnologique, ladite méthode comprenant les étapes suivantes :
a) hybridation d'une souche de levure S1 avec une souche de levure S2 ;
b) isolement des souches obtenues à l'étape a) ;
c) ensemencement des souches isolées à l'étape b) sur un moût;
d) étude de la cinétique de fermentation desdites souches ; et
e) sélection des souches présentant une cinétique de fermentation d'au moins 15%, de préférence au moins 30%, plus préférentiellement au moins 40% supérieure à la souche parente S1 et au moins 15%, de préférence au moins 30%, plus préférentiellement au moins 40% inférieure à la souche parente S2 sur le même moût que celui de l'étape c).

L'homme du métier connait plusieurs méthodes d'hybridation de souches de levure et saura utiliser une méthode adaptée pour hybrider la souche de levure S1 à la souche de levure S2. Dans le cas présent, les inventeurs ont découvert que la souche S1 ne peut pas produire de spores de type sexuel différencié a ou alpha ; cette souche est donc homothallique. Ainsi, un moyen d'hybridation de la souche S1 avec la souche S2 consisterait en une hybridation des spores de ces deux souches consécutivement à la dissection des tétrades de chaque souche. L'hybride naissant peut donc être le fruit du croisement d'une spore de S1 avec une spore de S2 ou bien le fruit du croisement d'une spore S1 avec une spore de S1 dont le type sexuel a changé au cours de la réplication. Les hybrides issus du croisement entre une spore de S1 et une spore de S2 sont par la suite spécifiquement sélectionnés par analyse des profils génétiques des hybrides naissant (par exemple par PCR Ty), et comparaison de ces profils aux profils génétiques des souches parentales.

Par « moût » on entend ici moût de raisin, c'est-à-dire la mixture obtenue après pression de raisins ou moût synthétique, c'est-à-dire reconstitué synthétiquement de manière à mimer au mieux la composition d'un moût de raisin naturel.

Plusieurs moûts peuvent être utilisés aux fins de la présente invention. A titre d'exemple, on peut citer le moût synthétique « vinicole Lesaffre » afin de présélectionner les levures, le moût synthétique standardisé de l'Organisme International de la Vigne et du Vin - OIV (RÉSOLUTION OIV-OENO 370-2012) afin de sélectionner les levures ou des moûts de raisins de cépages noirs concentrés en polyphénols issus ou non de macération préfermentaire à chaud afin de mettre les levures dans l'environnement pour lequel elles vont être destinées pour affiner leur sélection.

Dans un mode de réalisation, le test de fermentation est effectué sur un moût synthétique OIV.

Un « moût synthétique OIV » est un moût synthétique standardisé de l'Organisme International de la Vigne et du Vin - OIV (RÉSOLUTION OIV-OENO 370-2012) ajusté à 250g/l de sucres fermentescibles (pour obtenir un alcool potentiel de 15% v/v) avec une concentration initiale en azote de 200ppm.

Ainsi, selon un autre mode de réalisation, la présente demande concerne une méthode d'obtention d'une souche de levure œnologique, ladite méthode comprenant les étapes suivantes :
a) hybridation d'une souche de levure S1 avec une souche de levure S2 ;
b) isolement des souches obtenues à l'étape a) ;
c) ensemencement des souches isolées à l'étape b) sur un moût synthétique OIV;
d) étude de la cinétique de fermentation desdites souches ; et
e) sélection des souches présentant les trois caractéristiques suivantes :
   i) une cinétique de fermentation de 15 à 22 jours à une température de 24°C; et
   ii) une résistance à un degré alcoolique supérieur ou égal à 15% v/v; et
   iii) un besoin azoté inférieur ou égal à 200ppm.

Les levures ainsi sélectionnées sont capables de fermenter une grande quantité de sucres en utilisant une faible quantité d'azote, et ce même à un degré alcoolique élevé.

En outre, selon un autre aspect, l'invention concerne l'utilisation d'une souche de levure ou d'une levure telles que décrites ci-dessus pour la production de vin, particulièrement de vin rouge, encore plus particulièrement de vin à cépage noir très tannique. Dans un autre mode de réalisation, les souches de levure et levures obtenues/sélectionnées selon la présente invention sont utilisées pour la fermentation de vins rouges dits « de garde ».

Un « vin de garde » est un vin qui peut être conservé plusieurs années en cave et qui se bonifie en vieillissant, c'est le cas des vins grands crus de Bordeaux. Un vin de garde peut être gardé au moins cinq ans en cave.

### Brève description des figures

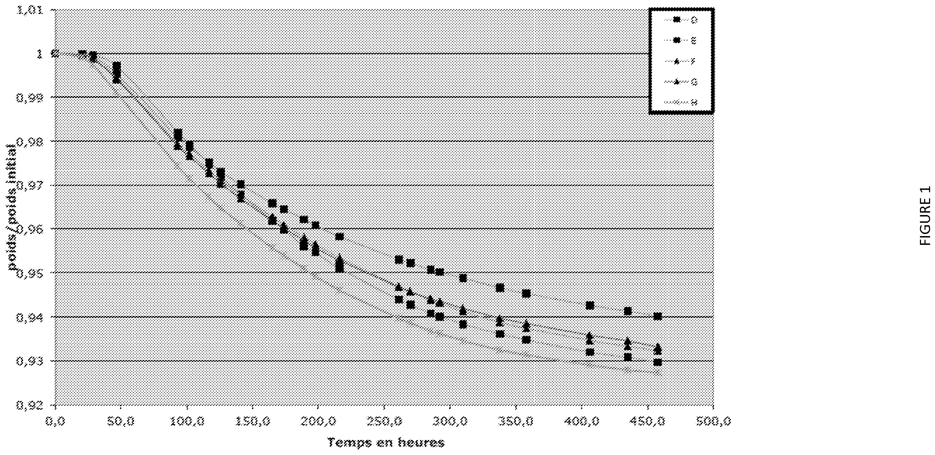

La **Figure 1** est un graphique représentant la cinétique de fermentation des souches 51-062 (H), 51-135 (F), S1 (D) et S2 (E) représenté par mesure du ratio poids/poids initial en fonction du temps.

### EXEMPLES

### Exemple 1 : Hybridation des souches S1 avec celles de S2.

### - Sporulation des deux souches parentales et développement des conditions opératoires de sporulation de la souche S1

Les conditions de sporulation ont été appliquées aux deux souches S2 et S1. Les taux de sporulation ont été calculés et sont présentés dans le Tableau 1.

**Tableau 1**

| **Souche** | **% ascospores** |
|---|---|
| Souche œnologique S2 | 13% |
| Souche œnologique S1 | 54% |

### - Isolement des spores par micro-manipulation et étude de l'auto-diploïdisation des spores issues des souches S2 et S1

Les conditions de sporulation ont été appliquées à plus grande échelle sur les deux souches œnologiques. Après traitement des asques et visualisation de tétrades au microscope, une dissection consistant à casser les tétrades, à prélever les spores puis à les déposer à des endroits précis de la gélose a été opérée. Le dénombrement des spores déposées et des spores ayant germé est présenté dans le Tableau 2 ci-dessous.

**Tableau 2**

| **Souche** | **Nombre tétrades disséquées** | **Nombre spores déposées** | **Nombre spores germées** | **Taux de Germination** |
|---|---|---|---|---|
| S2 | 50 | 200 | 73 | 36% |
| S1 | 33 | 132 | 95 | 72% |

Afin de pouvoir réaliser l'hybridation entre la souche S2 et S1, il faut obtenir pour chaque souche des spores de type sexuel bien différencié a ou alpha. Le signe sexuel de chaque spore obtenue précédemment a été étudié par PCR « mating type » (Tableau 3).

**Tableau 3**

| **Souche** | **Nombre spores germées** | **Signe a** | **Signe alpha** | **Signe a/alpha** | **Pas de résultat** |
|---|---|---|---|---|---|
| S2 | 39 | 20 | 14 | 4 | 1 |
| S1 | 95 | 0 | 0 | 95 | 0 |

Le Tableau 3 montre qu'il n'a pas pu être obtenu de spores de type sexuel différencié a ou alpha pour la souche S1. Il semblerait donc que la souche S1 soit homothallique. Dans ces conditions, il n'a pas été possible d'envisager un programme d'hybridation classique entre les souches S2 et S1.

### - Développement des conditions opératoires d'hybridation spécifiques au croisement S1 X S2, isolement des nouvelles souches et étude de leur profil génétique afin de sélectionner les hybrides issus du croisement entre la S1 et la S2.

Ne pouvant disposer d'une collection de spores de type sexuel a ou alpha pour la souche S1, les inventeurs ont entrepris de réaliser l'hybridation des spores de S1 et S2 consécutivement à la dissection des tétrades de chaque souche via le micromanipulateur. L'hybride naissant peut être ainsi le fruit du croisement d'une spore de S1 avec une spore de S2 ou bien le fruit du croisement d'une spore de S1 avec une spore de S1 dont le type sexuel a switché au cours de la réplication. Afin de sélectionner les hybrides issus du croisement entre une spore de S1 et une spore de S2 analysé les profils génétiques des hybrides naissants ont été analysés par PCR Ty, et ces profils ont ensuite été comparés à celui des souches parentales. Au final, 68 hybrides qui présentent des profils mixtes à différents degré par rapport au profil des souches parentales ont été conservés. Les qualités œnologiques de ces 68 souches ont ensuite été évaluées. Les souches 51-062 et 51-135 ont été sélectionnées comme suit.

### Exemple 2 : Développement des techniques de sélection des hybrides

Plusieurs techniques de sélection ont été utilisées:
a) Sélection en conditions synthétiques par l'étude de l'assèchement des sucres fermentescibles, la cinétique de fermentation et de la production de composés fermentaires d'intérêt des hybrides et des souches parentales sur moût synthétique vinicole Lesaffre (250g/L de sucres et 250ppm d'azote assimilable)= test A et moût synthétique OIV.
   Une première sélection, réalisée sur le critère cinétique, a permis de sélectionner 11 hybrides présentant de meilleures propriétés en termes d'assèchement du fructose que S1.
   Puis parmi ces hybrides, les souches 51-062 et 51-135 ont été sélectionnées car elles présentaient une cinétique moyenne entre la S1 et la S2 et en fin de fermentation, ces souches présentaient des productions de composés fermentaires intermédiaires mais différentes des deux souches parentes.
b) Sélection en conditions réelles par l'étude de la cinétique de fermentation et de la production d'esters sur moûts naturels des hybrides et des souches parentales :
   Le critère de sélection a été le suivi de la cinétique fermentaire, le contrôle des paramètres œnologiques et les analyses des principaux esters lors de microvinifications sur moût naturel de Cabernet Sauvignon. Il s'agit d'un moût obtenu par macération préfermentaire à chaud d'un cabernet sauvignon bien mûr. Le moût a été conservé à -60°C au congélateur à partir des vendanges de l'année.

Avant fermentation, le moût a été traité de la manière suivante : 1) Décongélation à température ambiante, 2) Clarification par centrifugation, 3) Séparation du moût en 5 lots homogènes.

Avant fermentation, le moût a été analysé par Spectroscopie infrarouge à transformée de Fourier + SO₂ par colorimétrie. Les résultats obtenus sont réunis dans le Tableau 4 ci-après :

**Tableau 4**

| **Glucos e + fructos e (g/L)** | **Acidité totale (g eq H2SO4 / L)** | **pH** | **Acide maliqu e (g/L)** | **Acide tartriqu e (g/L)** | **Acide citriqu e (g/L)** | **K (mg/L )** | **NH3 (mg/L )** | **NOP A (mg/L )** | **SO₂L (mg/L )** | **SO₂ T (mg/L )** |
|---|---|---|---|---|---|---|---|---|---|---|
| 246 | 4,30 | 3,3 4 | 3,38 | 1,8 | 0,66 | 670 | 42 | 103 | 0 | 0 |

Suite à l'analyse du moût, l'absence d'alcool et d'acide acétique avant fermentation ont également été constatés. 246g de Glucose + fructose correspondent à un degré probable de 14,6%vol (rendement de 16,83).

### - Préparations des levures sur tube gélosé : souches 51-135 (F) et 51-062 (H)

Préparation du milieu à incuber à Jour 0 : Extrait de levure 20g, Saccharose 100g, Solution minérale (100g MgSO4 + 100g KH2PO4 + Eau déminéralisée qsp 1L) 10 mL, Eau du robinet en qsp 1L.

A Jour 3 - inoculation des tubes à essai « A » (pour chaque souche testée): Une öse est prélevée sur la gélose est déposée dans 10ml de milieu à incuber 24 heures à 26°C.

A Jour 4 - préparation des tubes à essai « B » 1ml du tube A dans 9 ml de milieu à incuber 24 heures à 26°C

A Jour 5 : Verser chaque tube « B » dans 90ml de milieu à incuber 16 heures +/- 2 heures à 26°C avec une bonne agitation.

A Jour 6 : Mesure de la population avec une cellule de Thoma.

### Ensemencement des jarres (Tableau 5) :

**Tableau 5**

| **Préparation** | **Levures/ grand carré** | **Levures/ mlx1,6.10⁵** | **Levures/ 100mL** | **Volume ensemencé dans une jarre** | **Population estimée cell/L** |
|---|---|---|---|---|---|
| F (souche 51-135) | 152 | 2.10⁷ | 2.10⁹ | 100mL | 2.10⁸ |
| H (souche 51-062) | 173 | 3.10⁷ | 3.10⁹ | 100mL | 3.10⁸ |

Contrôle de la population dans les jarres (Tableau 6):

**Tableau 6**

| **Préparation** | **Levures/grand carré** | **Population cell/L** |
|---|---|---|
| F (souche 51-135) | 13 | 2.10⁹ |
| H (souche 51-062) | 16 | 3.10⁹ |

### - Préparation des levures sèches : souches D (souche S1) et E (souche S2) :

A Jour 6 : Hydratation des levures dans de l'eau stérile : 1g dans 10mL puis agitation 10 minutes aux Ultrasons, incubation à 30°C pendant 20 minutes, agitation 15 minutes aux Ultrasons, repos 20 minutes à température ambiante.

Les jarres ont été ensemencées à 2 ml de préparation dans IL de moût. La population a été vérifiée avec une cellule de Thoma : 10¹⁰ cell/L (Tableau 7) :

**Tableau 7**

| **Préparation** | **Levures/grand carré** | **Population cell/L** |
|---|---|---|
| D (souche S1) | 35 | 6.10⁹ |
| E (souche S2) | 34 | 5.10⁹ |

### - Suivi des fermentations

Les fermentations sont réalisées dans une pièce climatisée dont la température est maintenue à 18°C. Le suivi des cinétiques de fermentation est réalisé par pesées des jarres sur une balance à +/- 0,1g, 2 fois /jour environ. Les fermentations ont démarré à Jour 7 et ont été arrêtées à Jour 19.

Les résultats sont présentés dans la Figure 1.

En fin de fermentation : Soutirage et sulfitage aux environs de 60mg/L, mise en bouteille et conservation à 8°C.

### - Analyses sur vins finis

Analyse complète par Spectroscopie infrarouge à transformée de Fourier et SO2 par colorimétrie à J20 :

**Tableau 8**

| | **Titre alcoométri que volumique %v** | **Gluco se + fructo se (g/L)** | **Titre alcoométri que volumique total %v** | **Acidité totale (gH₂SO₄/ L)** | **Acide acétiq ue (g/L)** | **SO2 libre (mg/ L)** | **SO2 total (mg/ L)** | **pH** | **Acide maliq ue (g/L)** | **Acide Lactiq ue g/L** |
|---|---|---|---|---|---|---|---|---|---|---|
| **D** | 15,13 | 18 | 16,2 | 4,94 | 0,62 | 10 | 72 | 3,5 1 | 2,1 | 0 |
| **E** | 12,74 | 54 | 15,9 | 4,81 | 0,6 | 10 | 58 | 3,3 | 2,0 | 0 |
| | | | | | | | | 4 | | |
| **F** | 14,64 | 23 | 16 | 5,12 | 0,71 | 12 | 74 | 3,5 1 | 2,4 | 0 |
| **H** | 15,39 | 12,9 | 16,2 | 4,81 | 0,48 | 10 | 44 | 3,5 1 | 2,1 | 0 |

Selon la souche de levure, il est constaté :
- 0,5% volume d'écart sur le titre alcoométrique volumique total.
- Au bout de 19 jours, des sucres résiduels sont présents pour l'ensemble des souches.
- l'acidité totale varie de 4,69 à 5,12 g eqH2SO4/L
- l'acidité volatile varie de 0,48 à 0,71 g eqH2SO4/L
- La fermentation malolactique n'a pas démarré.

**Analyse de la couleur des polyphénols (Tableau 9)**

| | **Intensité colorante** | **Nuance** | **DO280** |
|---|---|---|---|
| **D** | 13,8 | 0,58 | 54 |
| **E** | 17,9 | 0,67 | 59 |
| **F** | 13,5 | 0,61 | 56 |
| **H** | 14,8 | 0,60 | 55 |

**Analyse des esters**

| **Echanti lllon** | **Ethyl butyr ate** (µg/L ) | **Ethyl-2-methylbut yrate** (µg/L) | **Ethyl Isobuty rate** (µg/L) | **Ethyl Levuli nate** (µg/L) | **Eth yl Isov ale rate** (µg/ L) | **Ethy l Hexa no ate** (µg/ L) | **Ethy l Octa no ate** (µg/ L) | **Ethy l Deca no ate** (µg/ L) | **Ethyl-2-Hydrox y-4-methylp enta noate** (µg/L) | **Isoa myl Acet ate** (µg/L ) |
|---|---|---|---|---|---|---|---|---|---|---|
| D | 170,2 | <5 | <5 | <20 | <5 | 254,5 | 349,8 | 339,3 | 168,3 | 609,4 |
| E | 128,6 | <5 | <5 | <20 | <5 | 253,3 | 466 | 473,8 | 84,5 | 484 |
| F | 219,9 | <5 | 5 | <20 | <5 | 279 | 441,3 | 366,1 | 201 | 436,2 |
| H | 276 | <5 | 7,1 | <20 | <5 | 386,5 | 567,5 | 447,6 | 211,5 | 523 |

### -Conclusion :

De façon générale, nous ne constatons pas de différence significative entre les résultats produits par la levure sèche D et ceux produits par les levures en tube gélosé.

| | **Levures sèches actives** | **Levures en tube gélosé** |
|---|---|---|
| **Titre alcoométrique total** | Pas de différence significative | |
| **Sucres résiduels** | Présence de sucres résiduels < 30 g/L Arrêt de fermentation | |
| **Acidité volatile** | Pas de différence significative sauf avec « H » = 0,48 | |
| **Fermentation malolactique** | Non faite | |
| **Intensité colorante** | 13,8 à 14,8 | |
| **Nuance** | 0,58 à 0,65 | |
| **DO280** | 54 à 56 | |

Les hybrides confirment une cinétique plus rapide que la S1 mais 2 groupes se forment :
- 51-135 montre une cinétique de fermentation intermédiaire par rapport à la S2 et la S1, des teneurs en sucres résiduels et en SO2 total (élevée donc négative) et une influence sur la couleur proches de la S2 et une production d'esters légèrement plus élevée mais proche des levures parentes.
- Le 51-062 montre une cinétique plus rapide que la S2 mais dont la pente est équivalente, une teneur en sucres résiduels, acidité volatile et SO2 plus faible que la S2 et une production d'esters plus importante que les souches parentes. Cette levure ne semble pas correspondre au critère cinétique mais présente des caractéristiques très intéressantes pouvant faire l'objet d'une étude approfondie.

## Revendications

1. Souche de levure obtenue par hybridation d'une souche S1, qui est la souche de levure déposée le 9 septembre 2015 à la Collection Nationale de Cultures de Microorganismes (CNCM) -Institut Pasteur, 25 rue du docteur Roux F75724 Paris sous le numéro I-5011, avec une souche S2, qui est la souche de levure déposée le 9 septembre 2015 à la Collection Nationale de Cultures de Microorganismes (CNCM) - Institut Pasteur, 25 rue du docteur Roux F75724 Paris (CNCM) sous le numéro I-5012, ladite souche de levure présentant, selon le test A, les caractéristiques suivantes :
- une cinétique de fermentation de 15 à 22 jours à une température de 24°C; et
- une résistance à un degré alcoolique supérieur ou égal à 15% v/v; et
- un besoin azoté inférieur ou égal à 200ppm,
où le test A correspond à un suivi de l'activité de fermentation de ladite souche de levure ensemencée à hauteur de 2.10⁶ UFC/ml sur le moût synthétique standardisé de l'Organisme International de la Vigne et du Vin OIV ajusté à 250g/l de sucres fermentescibles avec une concentration initiale en azote de 200ppm en fermentant à une température constante de 24°C.

2. Souche de levure selon la revendication 1, ladite souche étant la souche déposée le 5 mai 2015 à la Collection Nationale de Cultures de Microorganismes (CNCM) -Institut Pasteur, 25 rue du docteur Roux F75724 Paris CNCM en vertu du traité de Budapest, sous le numéro I-4975.

3. Souche de levure selon la revendication 1, ladite souche étant la souche déposée le 5 mai 2015 à la Collection Nationale de Cultures de Microorganismes (CNCM) -Institut Pasteur, 25 rue du docteur Roux F75724 Paris CNCM en vertu du traité de Budapest, sous le numéro I-4977.

4. Levure issue d'une souche de levure selon l'une quelconque des revendications 1 à 3.

5. Méthode d'obtention d'une souche de levure œnologique, ladite méthode comprenant les étapes suivantes :
a) hybridation d'une souche de levure S1, qui est la souche de levure déposée le 9 septembre 2015 à la Collection Nationale de Cultures de Microorganismes (CNCM) - Institut Pasteur, 25 rue du docteur Roux F75724 Paris sous le numéro I-5011, avec une souche de levure S2, qui est la souche de levure déposée le 9 septembre 2015 à la Collection Nationale de Cultures de Microorganismes (CNCM) - Institut Pasteur, 25 rue du docteur Roux F75724 Paris (CNCM) sous le numéro I-5012 ;
b) isolement des souches obtenues à l'étape a) ;
c) ensemencement des souches isolées à l'étape b) sur un moût synthétique standardisé de l'Organisme International de la Vigne et du Vin -OIV;
d) étude de la cinétique de fermentation desdites souches ; et
e) sélection des souches présentant les trois caractéristiques suivantes :
i) une cinétique de fermentation de 15 à 22 jours à une température de 24°C; et
ii) une résistance à un degré alcoolique supérieur ou égal à 15% v/v; et
iii) un besoin azoté inférieur ou égal à 200ppm.

6. Méthode d'obtention d'une souche œnologique selon la revendication 5, ladite méthode comprenant en outre une étape dans laquelle on sélectionne les souches présentant une cinétique de fermentation d'au moins 15%, de préférence au moins 30%, plus préférentiellement au moins 40% supérieure à la souche parente S1 et au moins 15%, de préférence au moins 30%, plus préférentiellement au moins 40% inférieure à la souche parente S2 sur moût synthétique OIV.

7. Utilisation d'une souche de levure selon l'une quelconque des revendications 1 à 3 ou d'une levure selon la revendication 4 pour la production de vin.

8. Utilisation selon la revendication 7, dans laquelle le vin est un vin rouge.

## Patentansprüche

1. Hefestamm, erhalten durch Hybridisierung eines S1-Stammes, der der Hefestamm ist, der am 9. September 2015 bei der Nationalen Sammlung von Kulturen von Mikroorganismen (CNCM) - Institut Pasteur, 25 rue du docteur Roux F75724 Paris, unter der Nummer I-5011 hinterlegt wurde, mit einem S2-Stamm, der der Hefestamm ist, der am 9. September 2015 bei der Nationalen Sammlung von Kulturen von Mikroorganismen (CNCM) - Institut Pasteur, 25 rue du docteur Roux F75724 Paris (CNCM) unter der Nummer I-5012 hinterlegt wurde, wobei der Hefestamm, gemäß dem Test A, die folgenden Eigenschaften aufweist:
- eine Fermentationskinetik von 15 bis 22 Tagen bei einer Temperatur von 24 °C; und
- eine Beständigkeit gegen einen Alkoholgehalt von mehr als oder gleich 15% v/v; und
- einen Stickstoffbedarf von weniger als oder gleich 200 ppm,
wobei der Test A einer Überwachung der Fermentationsaktivität des Hefestamms entspricht, ausgesät in einer Höhe von 2.10⁶ KBE/ml auf dem standardisierten synthetischen Most der Organisme International de la Vigne et du Vin OIV eingestellt auf 250 g/l fermentierbarer Zucker mit einer anfänglichen Stickstoffkonzentration von 200 ppm, wobei bei einer konstanten Temperatur von 24 °C fermentiert wird.

2. Hefestamm nach Anspruch 1, wobei der Stamm der Stamm ist, der am 5. Mai 2015 bei der Nationalen Sammlung von Kulturen von Mikroorganismen (CNCM) - Institut Pasteur, 25 rue du docteur Roux F75724 Paris CNCM gemäß dem Budapester Vertrag unter der Nummer I-4975 hinterlegt wurde.

3. Hefestamm nach Anspruch 1, wobei der Stamm der Stamm ist, der am 5. Mai 2015 bei der Nationalen Sammlung von Kulturen von Mikroorganismen (CNCM) - Institut Pasteur, 25 rue du docteur Roux F75724 Paris CNCM gemäß dem Budapester Vertrag unter der Nummer I-4977 hinterlegt wurde.

4. Hefe hervorgehend aus einem Hefestamm nach einem der Ansprüche 1 bis 3.

5. Verfahren zum Erhalten eines önologischen Hefestamms, wobei das Verfahren die folgenden Schritte umfasst:
a) Hybridisierung eines Hefestamms S1, der der Hefestamm ist, der am 9. September 2015 bei der Nationalen Sammlung von Kulturen von Mikroorganismen (CNCM) - Institut Pasteur, 25 rue du docteur Roux F75724 Paris unter der Nummer I-5011 hinterlegt wurde, mit einem Hefestamm S2, der der Hefestamm ist, der am 9. September 2015 bei der Nationalen Sammlung von Kulturen von Mikroorganismen (CNCM) - Institut Pasteur, 25 rue du docteur Roux F75724 Paris (CNCM) unter der Nummer I-5012 hinterlegt wurde;
b) Isolierung der in Schritt a) erhaltenen Stämme;
c) Aussaat der in Schritt b) isolierten Stämme auf einen standardisierten synthetischen Most der Organisme International de la Vigne et du Vin - OIV;
d) Untersuchung der Fermentationskinetik dieser Stämme; und
e) Auswahl der Stämme, die die folgenden drei Eigenschaften aufweisen:
i) eine Fermentationskinetik von 15 bis 22 Tagen bei einer Temperatur von 24 °C; und
ii) eine Beständigkeit gegen einen Alkoholgehalt von mehr als oder gleich 15% v/v; und
iii) einen Stickstoffbedarf von weniger als oder gleich 200 ppm.

6. Verfahren zum Erhalten eines önologischen Stammes nach Anspruch 5, wobei das Verfahren ferner einen Schritt umfasst, in dem die Stämme ausgewählt werden, die eine Fermentationskinetik von mindestens 15%, vorzugsweise mindestens 30%, bevorzugter mindestens 40% höher als der Elternstamm S1 und mindestens 15%, vorzugsweise mindestens 30%, bevorzugter mindestens 40% niedriger als der Elternstamm S2 auf synthetischem OIV Most aufweisen.

7. Verwendung eines Hefestamms nach einem der Ansprüche 1 bis 3 oder einer Hefe nach Anspruch 4 zur Herstellung von Wein.

8. Verwendung nach Anspruch 7, wobei der Wein ein Rotwein ist.

## Claims

1. A yeast strain obtained by hybridizing a strain S1, which is the yeast strain deposited on 9 September 2015 at the National Collection of Microorganism Cultures (Collection Nationale de Cultures de Microorganismes -CNCM) -Institut Pasteur, 25 rue du docteur Roux F75724 Paris under number 1-5011, with a strain S2, which is the yeast strain deposited on 9 September 2015 at the National Collection of Microorganism Cultures (Collection Nationale de Cultures de Microorganismes -CNCM) - Institut Pasteur, 25 rue du docteur Roux F75724 Paris under number 1-5012, said yeast strain presenting, according to test A, the following characteristics:
- fermentation kinetics from 15 to 22 days at a temperature of 24°C; and
- resistance with an alcoholic strength of more than or equal to 15% v/v; and
- a nitrogen requirement of less than or equal to 200ppm,
wherein test A corresponds to an analysis of the fermentation activity of said yeast strain seeded in the amount of 2.10⁶ CFU/ml on a synthetic must standardized by the International Organisation of Vine and Wine (Organisme International de la Vigne et du Vin - OIV) adjusted to 250g/l of fermentable sugars with an initial nitrogen concentration of 200ppm by fermenting at a constant temperature of 24°C.

2. The yeast strain according to claim 1, said strain being the strain deposited on 5 May 2015 at the National Collection of Microorganism Cultures (Collection Nationale de Cultures de Microorganismes -CNCM) - Institut Pasteur, 25 rue du docteur Roux F75724 Paris pursuant to the Budapest Treaty, under number 1-4975.

3. The yeast strain according to claim 1, said strain being the strain deposited on 5 May 2015 at the National Collection of Microorganism Cultures (Collection Nationale de Cultures de Microorganismes -CNCM) - Institut Pasteur, 25 rue du docteur Roux F75724 Paris pursuant to the Budapest Treaty, under number 1-4977.

4. Yeast isolated from a yeast strain according to any one of claims 1 to 3.

5. A method of obtaining an oenological yeast strain, said method comprising the following steps:
a) hybridizing a yeast strain S1, which is the yeast strain filed on 9 September 2015 at the National Collection of Microorganism Cultures (Collection Nationale de Cultures de Microorganismes -CNCM) -Institut Pasteur, 25 rue du docteur Roux F75724 Paris under number 1-5011, with a yeast strain S2, which is the yeast strain filed on 9 September 2015 at the National Collection of Microorganism Cultures (Collection Nationale de Cultures de Microorganismes -CNCM) - Institut Pasteur, 25 rue du docteur Roux F75724 Paris under number 1-5012;
b) isolation of the strains obtained in step a) ;
c) seeding of the strains isolated in step b) on a synthetic must standardized by the International Organisation of Vine and Wine (Organisme International de la Vigne et du Vin -OIV);
d) studying the fermentation kinetics of said strains ; and
e) selecting the strains presenting the following three characteristics :
i) fermentation kinetics from 15 to 22 days at a temperature of 24°C; and
ii) tolerance to an alcoholic strength of more than or equal to 15% v/v; and
iii) a nitrogen requirement of less than or equal to 200ppm.

6. The method of obtaining an oenological strain according to claim 5, said method also comprising a step in which strains presenting fermentation kinetics at least 15%, preferably at least 30%, more preferentially at least 40% greater than the parent strain S1 and at least 15%, preferably at least 30%, more preferentially at least 40% lower than the parent strain S2 on a synthetic OIV must are selected.

7. Use of a yeast strain according to any one of claims 1 to 3 or of a yeast according to claim 4 for producing wine.

8. The use according to claim 7, wherein the wine is red wine.
